Europäisches Patentamt

European Patent Office

Office européen des brevets

(11) Publication number: **0 299 682**
**A1**

## EUROPEAN PATENT APPLICATION

(21) Application number: 88306222.6

(22) Date of filing: 07.07.88

(51) Int. Cl.⁴: **C12N 15/00 , C07H 21/04 , C12N 9/08**

(30) Priority: 17.07.87 GB 8716938

(43) Date of publication of application:
18.01.89 Bulletin 89/03

(84) Designated Contracting States:
BE CH DE FR GB LI SE

(71) Applicant: **AMERSHAM INTERNATIONAL plc**
**Amersham Place**
**Little Chalfont Buckinghamshire HP7 9NA(GB)**

(72) Inventor: **Chiswell, David John**
**6 Halton Village Nr. Aylesbury**
**Buckinghamshire, HP22 5N5(GB)**
Inventor: **Ortlepp, Stephen Andrew**
**94 Hermitage Road St. Johns**
**Woking Surrey, GU21 ITQ(GB)**

(74) Representative: **Pennant, Pyers et al**
**Stevens, Hewlett & Perkins 5 Quality Court**
**Chancery Lane**
**London, WC2A 1HZ(GB)**

(54) DNA sequence coding for HRP enzyme.

(57) A synthetic DNA sequence which codes for the horseradish peroxidase C isoenzyme is described. The sequence includes a region coding for the isoenzyme of substantialy 927 base pain. The sequence includes spaced single restriction sites for the following enzymes in the following order starting at the 5´-end:
Sphl, Nhel, EcoRl, Pstl, BstEl, Narl, Ball, Rsal, Dral, Bcll, Ncol, EccRV.

Fig. 4

EP 0 299 682 A1

## DNA Sequence Coding for HRP Enzyme

Peroxidases are ubiqutious and abundant in nature performing tasks such as scavenging the potentially toxic free radical superoxide $O_2{}^-$. HRP is one of the most widely studied peroxidases and is extensively used as a detection reagent in a variety of systems ranging from immunoctyochemistry to Southern blots. It is a hydroxylase, catalysing the oxidation of various metabolites in the presence of hydrogen peroxide (Nobel and Gibson, 1970; Dordick et al., 1986).

Several studies have been performed to identify residues involved in the active site (Welinder and Mazza, 1977; Sakurado et al., 1986,; Shelnutt et al., 1986) and analyse different states of the enzyme (Sitter et al. 1986; Foote et al. 1987). In addition, although some physical (Shannon et al., 1966) and catalytic (Kay et al., 1967) properties of HRP have been described, little is known about the chemical mechanisms underlying its function.

Seven isoenzymes have been characterised in terms of their physical properties (Shannon et al., 1966) and their enzyme activities (Kay et al., 1967). These isoenzymes serve specialised biological functions: a) acidic, cell wall associated peroxidases of very high carbohydrate content, b) neutral and slightly basic peroxidases of lower carbohydrate content and c) very basic peroxidases of low carbohydrate content. Analysis of the conformation of different isoenzymes suggest that there are only small differences in their active sites (Strickland et al., 1968). Peroxidase C, dominates quantitatively among the horse radish isoperoxidases and its protein sequence has been determined (Welinder, 1979). It has an isoelectric point close to 9 and consists of a haemin prosthetic group, 2 $Ca^{++}$ and 308 amino acid residues, including 4 disulphide bridges in a single polypeptide chain that carries 8 neutral carbohydrate side chains. The molecular weight of the polypeptide chain is 33,922 (44,000 for the native protein including the carbohydrate side chains) (Welinder, 1979).

There is a need for a DNA sequence which can be used to generate the HRP C isoenzyme. For example, a cloned source of this dominant isoenzyme should enable substantial progress to be made in the study of the many outstanding problems of HRP enzymatic mechanisms. This invention concerns the synthesis and cloning of a gene specifying the isoenzyme sequenced by Welinder (1979).

This invention provides in one aspect a synthetic DNA sequence which codes for the HRP C isoenzyme or a conservative variant thereof or a peptide comprising at least one epitope or functional domain thereof. It will be appreciated that a large number of different DNA sequences can be designed, all of which will be capable in principle of coding for the HRP C isoenzyme. The sequence here described is substantially 940 base pairs and comprises a region coding for the HRP C isoenzyme of substantially 927 base pairs (specifying 309 amino acids).

It is well understood that the substitution of an amino acid residue of a protein or polypeptide by a different amino acid reside may (depending on the nature) and position of the amino acid) result in anything from a zero to a profound change in the properties of the protein or polypeptide. A conservative variant is HRP C isoenzyme, one or several amino acid residues of which have been replaced by one or several other amino acid residues, but without significantly changing the properties of the enzyme.

One particular property of the HRP C isoenzyme may be mentioned by way of example. HRP C isoenzyme is capable of taking part in a reaction resulting in the generation of enhanced luminescence from the catalysis of a substrate such as luminol in the presence of an enhancer of light output such as described in EPA 116454. Other HRP isoenzymes are less capable or incapable of showing this enhanced luminescence effect. A variant of the HRP C isoenzyme which is capable of shwoing this enhanced luminescence effect is deemed a conservative variant.

The synthetic DNA sequence may code for a peptide comprising at least one epitope or functional domain of the enzyme. It is believed that active amino acids in the enzyme include arginine at position 183 and tyrosine at position 185 (of the sequence of Welinder). Among peptides which, by virtue of including these two amino acids, comprise at least one epitope or functional domain of the enzyme, may be mentioned the following:-

i) Arg Leu Tyr, and

ii) Gly His Thr Phe Gly Lys Asn Gln Cys Arg Phe Ile Met Asp Arg Leu Tyr Asn Phe Ser Asn Thr Gly Leu Pro.

For ease of manipulation, the synthetic DNA sequence may include single restriction sites for a variety of commercially available restriction enzymes. The most 5′ restriction site is preferably recognised by an enzyme such as SphI which contains an initiation codor (AUG) within its recognition sequence allowing manipulation of the DNA so that it encodes an initiation codon or not (removal of an overhang). The most 3′ restriction site is preferably recognised by an enzyme such as EcoRV, which allows blunt end ligation into

2

most vectors. The sequence preferably includes spaced single restriction sites for:

Sphl, EcoRl, Narl, Dral, Ncol and EcoRV.

More preferably, this sequence includes spaced single restriction sites for the following enzymes in the following order starting at the 5' end:

Sphl, Nhel, EcoRl, Pstl, BstEl, Narl, Ball, Rsal, Dral, Bcll, Ncol, EcoRV.

The invention also includes vectors containing the synthetic DNA sequence, which vectors may be based on the plasmid pUC9 or the plasmid pAM18.

The invention also includes a method of making the HRP C isoenzyme or a conservative variant thereof or a peptide comprising at least one epitope or functional domain thereof, which method comprises introducing a vector as defined into a host cell and recovering the isoenzyme or peptide therefrom.

In the following text, certain abbreviations are used:

Ap, ampicillin; bp, base pair(s); CIP, calf intestinal alkaline phosphatase; EtdBr, Ethidium bromide; HRP, horse radish peroxidase; IPTG, isopropylthiogalactoside; kb kilobases; LB, Luria broth; LMP, low melting temperature agarose; Pollk, DMA polymerase I (large fragment); RF, replicative form DNA; XGAL, 5-bromo-4chloro-indolyl-beta-D-galactoside.

Reference is directed to the accompanying drawings.

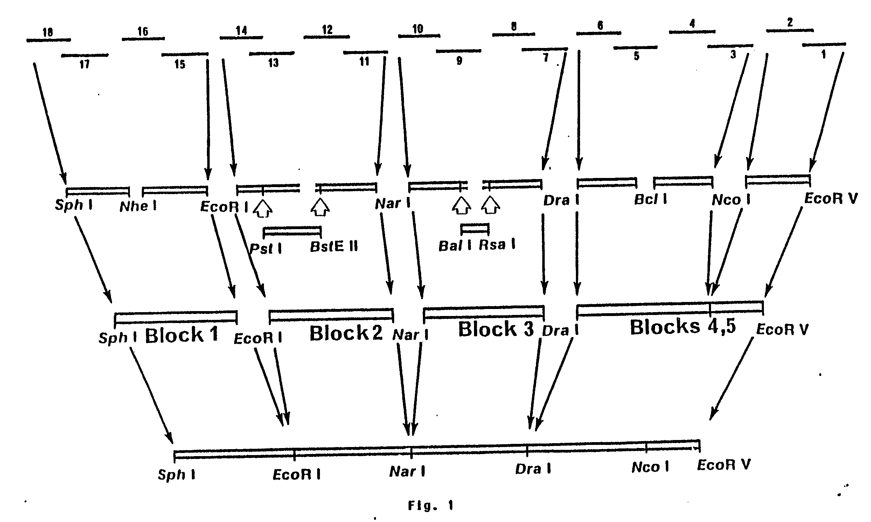

Figure 1. Assembly of synthetic HRP coding sequence. The eighteen oligonucleotides (top row) were cloned in pairs into M13. After confirmation of their sequence they were subcloned in pUC9. The four blocks were assembled from these subsections. In the cases of blocks 2 and 3 the middle sections of the blocks were obtained by annealing and filling in oligonucleotides 11-14 and 7-10 respectively and digesting the resulting doublestranded DNA with either Pstl and BstEll (block 2) or Ball and Rsal (block 3). These DNA fragments were then used in three way ligations to complete their respective blocks. Blocks 1 and 4 contain a Nhel (block 1) or a Bcll (block 4) site in the overlap between the centre oligonucleotides and could therefore be assembed by direct ligation. In the case of block 4 this construction was carried out in a dam⁻ host. Next, block 3 was ligated to blocks 4,5 and a final three way ligation resulted in the complete coding sequence.

Figure 2. Oligonucleotides synthesised to generate complete HRP gene. The gaps on both strands indicate the stretches of DNA that were filled in using Pollk. Included are the landmark restriction sites used in the final assembly of the gene.

Figure 3. Construction of blocks 2 and 3.

(A) Oligonucleotides 13,14 and 11,12 were cloned in pUC9 generating plasmids pSA220 and 221 respectively. pSA220 was cut with Pstl and HindIll and purified from LMP agarose. pSA221 was cut with BstEll and HindIll and the resulting 110bp fragment was also purified from LMP agarose. Oligonucleotides 11-14 were annealed and filled in as described, cut with Pstl and BstEll and a three way ligation resulted in pSA227, carrying the complete block 2.

(B) Oligonucleotides 9,10 and 7,8 were cloned in pUC9 generating plasmids pSA222 and pSA223 respectively. pSA222 was cut with Ball and HindIll and purified from LMP agarose. pSA223 was cut with Rsal and HindIll and the fragment carrying the HRP sequence was purified from LMP agarose. Oligonucleotides 7-10 were annealed and filled in as described, cut with Ball and Rsal and a three way ligation resulted in pSA228. The correct orientation of the centre section was established by digestion with Ball.

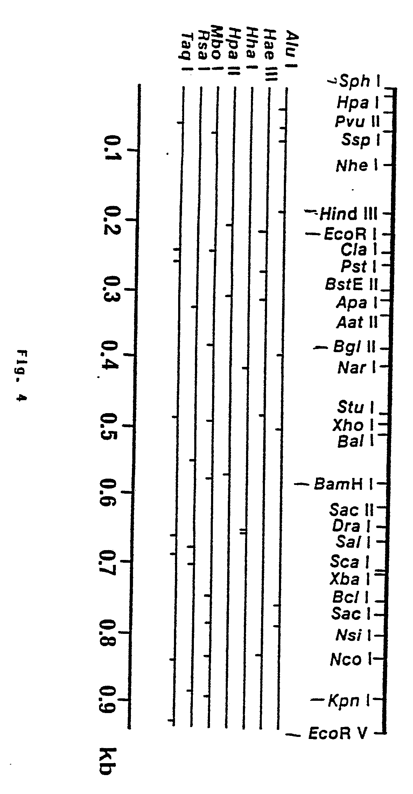

Figure 4. Restriction map of synthetic HRP gene. There are additional single restriction sites present that have not been shown (the figure in brackets denoted the position of the restriction site within the gene): Bgll(339), BssHII(652), HgiAl(750), Mlul(154), Nael(339), Nrul(58), Pvul(245), Rsrll(84), SfaNl(109), Smal-(226), SnaBl(673), Spel(788), Xmnl(283).

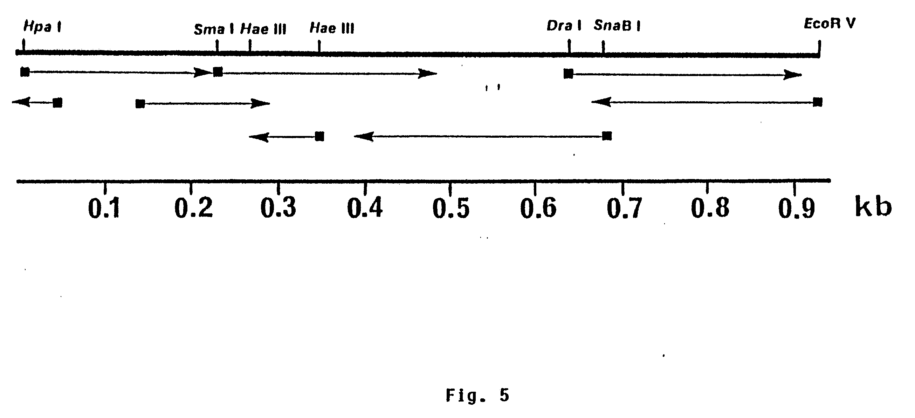

Figure 5. Sequencing strategy for the 940bp insert in pSA233. The extreme 5' end of the insert was sequenced directly from pSA233 using one of the 66mers (oligonucleotide 17) as the primer as well as in M13.

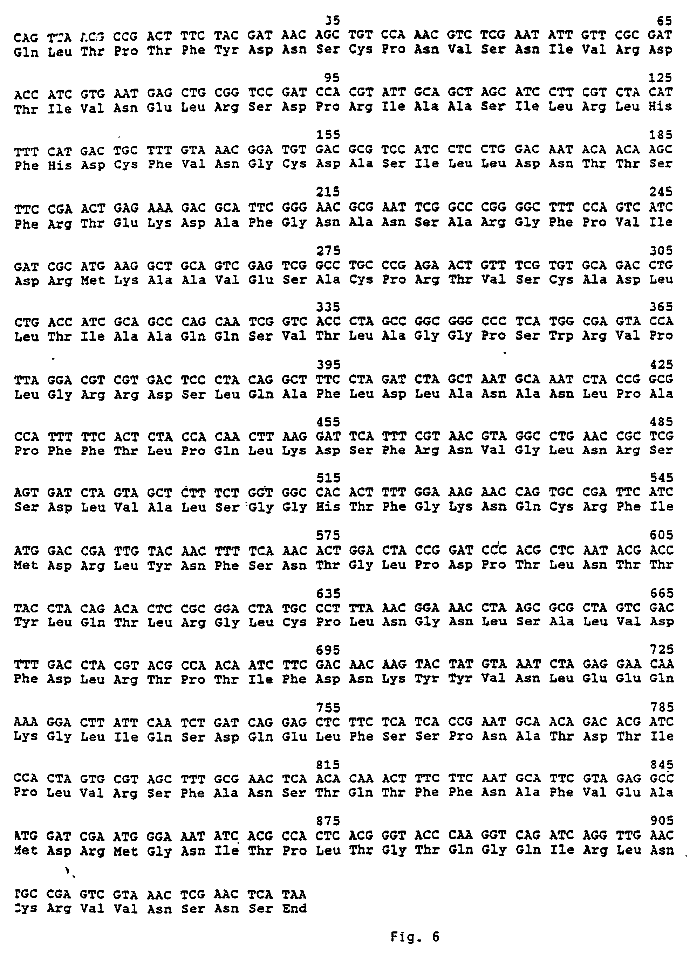

Figure 6. DNA and amino acid sequence of the HRP isoenzyme C.

There follows a description of the synthesis and cloning of the gene, in which reference is made to the figures.

## (a) Gene Design

Unlike the strategies used in previously reported syntheses of genes (Khorana, 1979, Gearing et al., 1985; Ferretti et al., 1986) here the 5'-3' polymerase activity of PolIk has been relied on to generate double stranded DNA from partially overlapping oligonucleotides. This approach minimises the number of bases having to be synthesised. A number of criteria were considered when designing the gene:

i) incorporation of a large number of single restriction sites. In fact, there are single restriction sites for 42 different commercially available enzymes. They are spaced so as to maximise the ease with which further manipulations can be carried out. This is true for both six and four cutters. At the same time a number of sites were omitted (e.g. NdeI). This will make it simpler to generate constructs requiring manipulation of the vector plasmid.

ii) care was taken to remove sequences that could introduce instability.

iii) the base distribution was normalised and codon usage was adjusted so that there is no preference for any particular codon.

iv) what is believed to be the active site of the protein is specified by block 3 making up the gene. This allows random mutagenesis of this block alone followed by substitution into the complete gene.

v) the most 5' restriction site is recognised by SphI, allowing manipulation of the DNA so that it encodes an initiation codon (AUG) or not (removal of the 3' overhang).

vi) the most 3' restriction site is recognised by EcoRV. This allows blunt end ligation into most vectors.

## (b) Cloning of Oligonucleotides

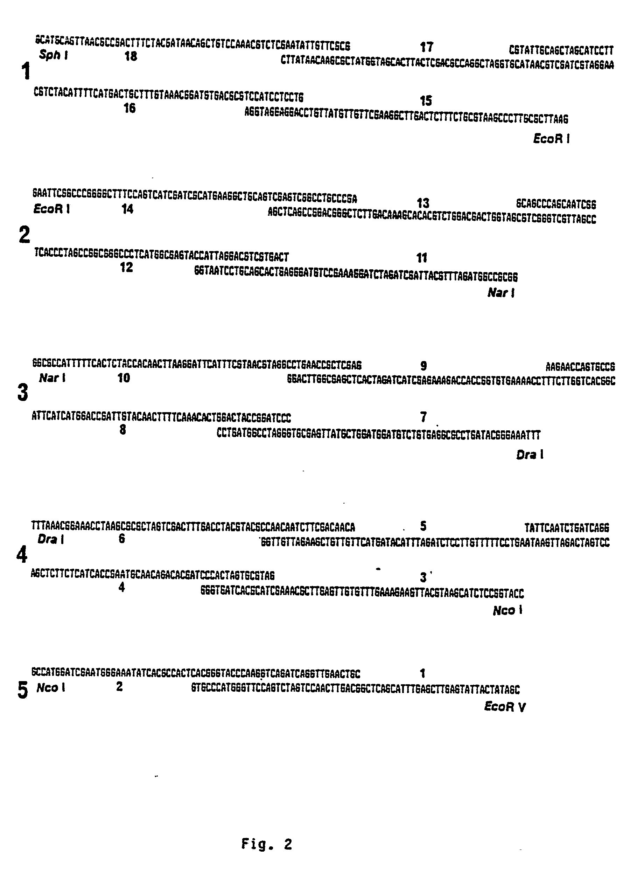

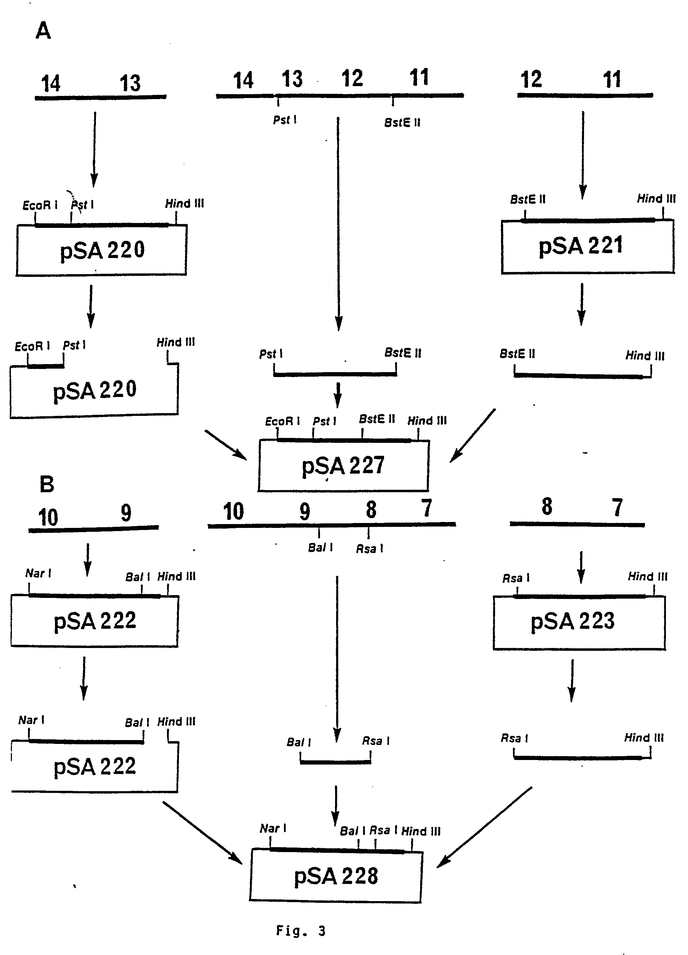

The strategy used to assemble the complete coding region for a HRP protein is detailed in Figure 1. The original strategy was to generate four blocks of four oligonucleotides each and one of two oligonucleotides and clone these into pUC9. These could then be cut out with appropriate restriction enzymes and assembled into the complete HRP gene. The oligonucleotides were therefore designed so that the 5' and 3' oligonucleotides in each block had a convenient restriction site allowing ligation to the neighbouring block, whereas within a block the oligonucleotides would anneal at complementary regions of between 11 and 34bp. The average region of complementarity was 18bp (Figure 2). After annealing, PolIk could then fill in the gaps and after kinasing the blocks could be cloned (Figure 3). This strategy however was unsuccessful and an alternative assembly had to be carried out: The oligonucleotides were annealed in pairs and cloned into M13. After cloning the 18 oligonucleotides in pairs into M13 the inserts were sequenced. In contrast to others (McClain et al. (1986)) a very low percentage of mutant bases was found in the synthetic DNA sequence: only 4 out of the 1189 bases synthesised. Plaques harbouring phage with inserts specifying the correct sequences were selected, RF DNA was prepared, digested as appropriate and inserts were purified from LMP gel (see Table 1 for list of plasmids).

## (c) Assembly of the five blocks

Blocks 1 and 4 contain single restriction sites within the overlaps of the central two oligonucleotides. In the case of block 1 this is a NheI site and in the case of block 4 a BclI site. To assemble block 1, oligonucleotides 15,16 were excised from M13 by digestion with NheI and EcoRI and oligonucleotides 17,18 were excised by digestion with NheI and SphI. In a three way ligation to pAM18 cut with SpHi and EcoRI plasmid pSA226 specifying the complete block 1 was generated. Blocks 4 and 5 were assembled in a single ligation step: oligonucleotides 3,4 and 5,6 were subcloned into pUC9 generating pSA224 and pSA225. As BclI cuts only unmethylated DNA the plasmids were transformed into NT104. Rapid DNA preparations were cut with HindIII and BclI (pSA224) and NCoI and BclI (pSA225). HindIII cuts in the polylinker region. Oligonucleotides 1,2 had been excised from M13 by digestion with NcoI and EcoRI. A four way ligation into pUC9 cut with HindIII and EcoRI resulted in a 302bp insert comprising the complete block 4 and block 5 generating plasmid pSA229. Construction of remaining blocks 2 and 3 is described in Figure 3. In light of the fact that their assembly relied on the ability to anneal all four oligonucleotides to generate the central links the original failure to clone complete blocks of four annealed oligonucleotides remains unexplained.

(d) Assembly of the Complete Coding Sequence of the HRP Gene

pSA228 was digested with EcoRI (cutting in the polylinker proximal to the Narl site and Dral. pSA229 was digested with Dral and EcoRI (cutting in the polylinker proximal to the EcoRV site). The 203bp fragment specifying block 3 and the 302bp fragment specifying blocks 4 and 5 were purified from LMP agarose. A three way ligation into pUC9 cut with EcoRI and dephosphorylated resulted in pSA230. To confirm that this plasmid specifies blocks 3,4 and 5 it was cut with StuI, NsiI and KpnI which cut blocks 3,4 and 5 respectively. All three sites were present (not shown). Finally, pSA226 was digested with SphI and EcoRI and dephosphorylated. pSA227 was digested with Narl, dephosphorylated and cut with EcoRI. pSA230 was cut with Narl and EcoRI. Linear pSA226, the 203bp fragment specifying block 2 and the 514bp fragment specifying blocks 3,4 and 5 were purified from LMP agarose. A three way ligation resulted in pSA233, expected to specify the complete coding sequence for HRP. The codon distribution and breakdown of the protein into its constituent amino acids is detailed in Table II.

(e) Diagnostic Restriction Mapping of Assembled Gene

pSA233 was digested with a number of restriction enzymes to confirm that it contained an insert of the correct size and that the cassettes had been ligated in the correct order and orientation. Double digestion with SphI and EcoRV released a fragment of approximately 940bp, while double digestions with SphI and EcoRI, BgIII, SacII and NcoI released fragments of 720 and 220, 400, 620 and 845bp respectively. The extra fragment in the SphI/EcoRI double digest derives from the way pSA233 was constructed, with an extra EcoRI site in the polylinker region downstream from the 3' terminal EcoRV site. Digests with a further 27 enzymes that cut the HRP gene once confirmed that the insert in pSA233 contained all the constituent blocks. An extensive restriction map is shown in Figure 4.

(f) Sequencing of HRP gene

The HRP gene was excised from pSA233 by digestion with SphI and EcoRV and purified from LMP agarose. The insert was cut with Dral, HaeIII, HpaI, SnaBI or SmaI and subcloned in SmaI cut, dephosphorylated M13 mp8. The extreme 5' end of the gene was sequenced using linearised pSA233 as the template and oligonucleotide 17 as the sequencing primer. The sequencing strategy is shown in Figure 5. Complete agreement of the cloned sequence with the synthesised sequence confirmed that the insert in pSA233 specifies the HRP protein (Figure 6).

g) Stabilisation of Blocks and Complete Gene

A number of problems were encountered with the stability of both individual blocks as well as the complete gene. Four problems in particular required solving: i) persistent loss of the BgIII site; ii) inability to use the BamHI site for insertion of the central region of block 3; iii) initial partial digestion at the EcoRV site, leading to deletion of that site; iv) deletion of either the NcoI site and retention of the KpnI site or loss of the KpnI site and retention of the NcoI site. Use of a long wave UV lamp when isolating DNA from LMP agarose gels and forced cloning and selective dephosphorylation making it unnecessary to purify fragments at all solved the BgIII and EcoRV problems. Reisolation of oligonucleotides 3 and 4 solved the NcoI/KpnI problems without answering the question why the previous constructs had failed. The apparent inability to clone into the BamHI site in block 3 was remedied by using a nearby RsaI site to insert the central fragment (see Figure 1). Once the block was assembled, no more instability was observed. Only one instance of a 250bp deletion was observed once the complete gene had been assembled in pAM18. Numerous other isolates however have proved to be perfectly stable and several large scale preps, both amplified and non-amplified have been prepared and no more instability has been observed. The correct DNA sequence was derived from DNA purified from such a bulk preparation. To test the stability of the HRP gene in a different vector, it was subcloned as a SphI-EcoRV fragment into pUC18 cut with SphI and SmaI, generating pSA244. This plasmid is stable and no deletions have been observed so far. This plasmid will become the basic plasmid for generation of expressed and secretion derivatives.

## (h) Expression of HRP Gene in E.coli

A DNA fragment specifying a promoter and ribosome binding site was ligated upstream from the HRP gene generating plasmid pSA 235. The gene can be induced by addition of IPTG to the growth medium. Colonies are stained with a dye (N,N,N',N'-tetramethylphenylene diamine) which is oxidized in the presence of HRP to give a dark blue colour. Only colonies expressing the HRP gene turn blue, control colonies remain colourless. When cell lysates from colonies expressing the HRP gene and from colonies not expressing the HRP gene were run on a gel and the gel was stained with the dye, a blue band appeared only in the lane containing the HRP lysate. This provides evidence for expression of an active HRP in E.coli

A plasmid, pSA 247, was constructed, this plasmid is equivalent pSA 235 with the addition of the LacI<sup>q</sup> gene. This plasmid was expressed in E.coli and specified production of a protein of molecular mass 33KDa, as expected from the non-glycosylated form of isoenzyme C. This protein was also immunoreactive with an anti-HRP antibody. The partially purified protein exhibits enzymic properties expected of HRP.

There follows a description of materials and methods used in the production of the new gene.

## (a) Bacterial strains, plasmids and culture conditions

E. coli JM101 (supE, thi, $\Delta$ (lac-proAB), [F',traD36, proAB$^+$, lacI$^q$, lacZ$\Delta$M15]) was the major host strain used throughout these experiments and was routinely grown on minimal medium (Maniatis et al. (1982). Cells containing recombinant plasmids were routinely grown on LB with ampicillin (100$\mu$g/ml), X-GAL (0.004%), IPTG ($4 \times 10^{-5}$M) and agar (1.8%) added as appropriate. For experiments requiring digestion of DNA with BclI, NT104 (dam$^-$ dcm$^-$) was used. Strains were made competent and transformed by the method of Mandel and Higa (1970). Competent cells were routinely frozen and stored at $^-70^{\bullet}$C in 20% glycerol. Plasmids used are shown in Table I. pAM18 and M13 (SmaI cut and dephosphorylated) were supplied by Amersham International.

## (b) Plasmid isolation

Plasmids and M13 RF were prepared by a modified version of the method of Holms and Quigley (1981). Small scale rapid DNA preparations were carried out by adding a loopful of bacteria to 100$\mu$l of STET buffer (8% Sucrose, 0.05M Tris-HCl, pH 8.0, 0.05M EDTA, pH 8.0, 5% (v/v) Triton-X-100) to which 1mg/ml of lysozyme (Sigma) was added. After 2 minutes at room temperature the lysed cells were boiled for 1 minute and spun in an Eppendorf microfuge for 4 minutes. Pellets were removed with a tooth pick and discarded. 10$\mu$l of 3M sodium acetate was added to the supernatant followed by 2 volumes of ethanol. After mixing, tubes were spun immediately in an Eppendorf centrifuge for 4 minutes, the supernatant was removed and pellets were dried under vacuum. Using this faster modification, up to 5$\mu$g of plasmid DNA can be extracted from each of 18 preparations in less than 30 minutes. The DNA was resuspended in 50$\mu$l water and 10$\mu$l were used for each digestion. RNAase was added with gel loading buffer (0.1% Bromophenol blue, 0.1% Xylene cyanol, 15% sucrose) just prior to loading on a gel. Large scale preparations were prepared by the method of Birnboim and Doly (1979) and purified by CsCl-centrifugation.

## (c) DNA Manipulations

Restriction enzymes were generally supplied by Amersham or, when not available, purchased from New England Biolabs and used according to manufacturer's instructions. DNA ligase, PolIk and T4 polynucleotide kinase were supplied by Amersham. DNA ligase and polynucleotide kinase were used according to manufacturer's specifications. CIP was purchased from Boehringer, 20 units were incubated with 1ug DNA for 1 hour at 37$^{\bullet}$C. DNA samples were electrophoresed in horizontal 0.7% agarose gels in TBE buffer (0.067M Tris, 0.022M boric acid, 0.002M EDTA, pH 8.8). Bands were visualised on a UV transilluminator after staining gell in 2$\mu$g/ml EtdBr and photographed on Polaroid 667 film. LMP agarose was purchased from BRL. DNA fragments were extracted by adding an equal volume of water to the gel slice and melting it at 70$^{\bullet}$C for 5 minutes. Two phenol and one chloroform isoamyl alcohol (24:1 v/v) extraction were followed by an ethanol precipitation. The purified DNA was resuspended in water to a concentration of approximately 0.1$\mu$g/ml.

## (d) Oligonucleotide Synthesis

17 66mers and 1 67mer were synthesised on an Applied Biosystems 381A synthesiser using phosphoramidite chemistry (Beaucage and Caruthers, 1981) on 0.20μM controlled pore columns. Both methyl- (Daub and van Tamelen, 1977) and beta-cyanoethyl phosphoramidites (Sinha et al. 1984) were used. Crude beta-cyanoethyl oligonucleotides were cleaved off and the solid support and fully deprotected by incubation in 1.5ml concentrated ammonium hydroxide at 55°C for 12 hours. Oligonucleotides synthesised with methyl-phosphoramidites were deprotected by incubation in thiophenol for 30 minutes. The crude oligonucleotides were cleaved from the solid support by four consecutive 15 minute treatments with concentrated ammonium hydroxide. Purification of crude oligonucleotides was carried out according to the Applied Biosystems user bulletin 13 (1984). After lyophilisation the oligonucleotides were electrophoresed through 15% polyacrylamide/urea gels (Maniatis et al., 1982), the gels were stained in 2μg/ml EtdBr and the correct size bands were located using a handheld long range UV lamp. They were excised and purified as described by Maniatis et al., (1982). Purified oligonucleotides were resuspended in TE-buffer (0.01M Tris-HCl, pH 7.5, 0.001M EDTA) to a concentration of 0.5μg/ml.

## (e) Annealing and cloning of oligonucleotides

Single stranded oligonucleotides (2.5μg each) were mixed in pairs as appropriate at a 1:1 molar ratio in 1x Klenow buffer (0.05M Tris-HCl, pH 7.5, 0.05M NaCl 0.01M MgCl₂) in a total volume of 20μl, boiled for 3 minutes and slowly cooled to 30°C. The annealed mixture was converted to double stranded DNA by incubation with 3 units PolIk and all four nucleoside triphosphates (0.5mM) in 50μl at 30°C for 2 hours. To obtain the centre fragments for blocks 2 and 3, all four oligonucleotides constituting a block were mixed and annealed. 6 units of PolIk were added and the volume of the reaction as well as the concentration of dNTPs was also doubled. Double stranded DNA was purified from LMP agarose gels, kinased and ligated to M13 mp8 cut with SmaI and dephosphorylated. The ligation mix was used to transform JM101 cells. White plaques were isolated, the DNA was purified and used as template in dideoxy sequencing reactions.

## (f) Nucleotide Sequence Determination

Sequencing reactions were carried out according to the dideoxy chain termination procedure of Sanger et al., (1977). Amersham's M13 cloning and dideoxy sequencing kits were used throughout. Double stranded plasmid sequencing was carried out on heat denatured templates essentially as described by Wallace et al. (1981).

## References

Beaucage, S.C. and Caruthers, M.H.: Deoxynucleoside phosphoramidites - A new class of key intermediate for deoxypolynucleotide synthesis. Tetradedon Letters 22 (1981) 1859-1862.

Birnboim, H.C. and Doly, J.: A rapid alkaline extraction procedure for screening recombinant plasmid DNA. Nucl. Acids Res. 7 (1979) 1513-1523.

Daub, G.W. and van Tamelen, E.E.:Synthesis of oligoribonucleotides based on the facile cleavage of methyl phosphotrisester intermediates. J. Amer. Chem. Soc. 99 (1977) 3526-3528.

Dordick, J.S., Klibanov, A.M. and Marletta, M.A.: Horseradish peroxidase catalysed hydroxylations: Mechanistic studies. Biochemistry 25 (1986), 2946-2951.

Ferretti, L., Karnick, S.S., Khorana, H.G., Nassal, M. and Oprian D.D.: Total synthesis of a gene for bovine rhodopsin. Proc. Natl. Acad. Sci. USA 83 (1986) 599-603.

Foote, N., Gadsby, P.M.A., Field, R.A., Greenwood, C. and Thomson, A.J.: A comparison by magnetic circular dichroism of compound X and compound II of horseradish peroxidase. FEBS Lett 214 (1987) 347-350.

Gearing, D.P., McMullen, G.L. and Nagley, P: Chemical synthesis of a mitochondrial gene designed for expression in the yeast nucleus. Biochemistry International 10 (1985) 907-915.

Holmes, D.S. and Quigley, M: A rapid boiling method for the preparation of bacterial plasmids. Anal. Biochem. 114 (1981), 193-197.

Hosoda, J., Takashi, W., Oe, T., Tsukamoto, R. and Nambara, T.: A comparison of chromogenic substrates for horseradish peroxidase as a label in steroid enzyme immunoassay. Chem. Pharm. Bull, Tokyo 34 (1986) 4177-4182.

Kay, E., Shannon, L.M. and Lew, J.Y.: Peroxidase Isozymes from horseradish roots II. Catalytic properties, J. Biol. Chem. 242 (1967) 2470-2473.

Kuhlmann, W.D. and Peschke, P.: Glucose oxidase as label in histological immunoassays with enzyme amplification in a two step technique: immobilised horseradish peroxidase as secondary system enzyme for chromogen oxidation, Histochemistry 85 (1986) 13-18.

Khorana, H.G.: Total synthesis of a gene. Science 203 (1979) 614-625 Mandel. M. and Higa, A.: Calcium dependent bacteriophage DNA infection. J. Mol. Biol. 53 (1970) 154-161.

Maniatis, T.. Fritsch, E.F. and Sambrook, J.: Molecular Cloning. A Laboratory Manual. Cold Spring Harbour Laboratory, Cold Spring Harbour, NY, 1982.

McClain, W.A., Foss, K., Mittelstadt, K.L. and Schneider, J.: Variants in clones of gene-machine synthesised oligodeoxynucleotides Nucl. Acids Res. 14 (1986) 6771.

Nobel, R.W. and Gibson, Q.H.: The reaction of ferrous horseradish peroxidase with hydrogen peroxide. J. Biol. Chem. 245 (1970) 2409-2413.

Porter, L.L. and White, E.L.: Synaptic connection of callosal projection neurons in the vibrissal region of mouse primary motor cortex: An electron microscopy/horseradish peroxidase study. J. Comp. Neurol. 248 (1986) 573-588.

Sakurada, J., Takahashi, S. and Hosaya, T.: Nuclear magnetic resonance studies on the spacial relationship of aromatic donor molecules to the heme iron of horseradish peroxidase. J. Biol Chem. 261 (1986) 9657-9662.

Sanger, F., Micklen, S. and Coulson, A.R.: DNA sequencing with chain-terminating inhibitors. Proc. Natl. Acad. Sci. USA 74 (1977) 5463-5467.

Shannon, L.M., Kay, E. and Lew, J.Y.:Peroxidase Isozymes from horseradish roots I. Isolation and physical properties. J. Biol. Chem. 241 (1966) 2166-2172.

Shelnutt, J.A., Alden, R.G. and Ondrias, M.R.: Heme linked ionisation in horseradish peroxidase detected by Raman Difference Spectroscopy. J. Biol. Chem. 261 (1986) 1720-1723.

Sinha, N.D., Biernat, J., McManus, J. and Koster, H.: Polymer support oligonucleotide synthesis XVIII[2]: use of beta-cyanoethyl-N,N-dialkylamino-/N-morpholino phosphoramidite of deoxynucleosides forn the synthesis of DNA fragments simplifying deprotection and isolation of the final product. Nucl. Acids Res. 12 (1984) 4539-4557.

Sitter, A.J. Reczek, C.M. Tenner, J.: Comparison of heme structures of horseradish peroxidase compounds X and II by resonance Raman spectroscopy. J. Biol. Chem. 261 (1986) 8638-8642.

Strickland, E.H., Kay, E., Shannon, L.M. and Horwitz, J.: Peroxidase Isozymes from horseradish roots III. Circular dichroism of isoenzymes and apoisoenzymes. J. Biol. Chem. 243 (1968) 3560-3565.

Vieira, J. and Messing, M.J.: the pUC plasmids, and M13mp7- derived system for insertion mutagenesis and sequencing with synthetic universal primers. Gene 19 (1982) 269-276.

Wallace, R.B., Johnson, M.J.Suggs, S.Y., Myoshi K., Bhatt, R. and Itakura, K.: A set of synthetic primers for DNA sequencing in the plasmid pBR322. Gene 16 (1981) 21-26.

Welinder K.G.: Amino acid sequence studies of horseradish proxidase. Eur. J. Biochem. 96 (1979) 483-502.

Welinder, K.G. and Mazza, G.: Amino acid sequences of heme linked histidine containing peptides of five peroxidases from Horse Radish and Turnip. Eur. J. Biochem. 73 (1977) 353-358.

Table I

| List of Plasmids | | |
|---|---|---|
| Plasmid | Description | Reference |
| pUC9 | Ap$^R$ | Vieira and Messing (1982) |
| pAM18 | Ap$^R$ | Amersham Research News Vol.1,4 |
| pSA220 | Oligonucleotides 13,14 in pUC9 | This work |
| pSA221 | Oligonucleotides 11,12 in pUC9 | This work |
| pSA222 | Oligonucleotides 9,10 in pUC9 | This work |
| pSA223 | Oligonucleotides 7,8 in pUC9 | This work |
| pSA224 | Oligonucleotides 5,6 in pUC9 | This work |
| pSA225 | Oligonucleotides 3,4 in pUC9 | This work |
| pSA226 | Block 1 in pAM18 | This work |
| pSA227 | Block 2 in pUC9 | This work |
| pSA228 | Block 3 in pUC9 | This work |
| pSA229 | Blocks 4,5 in pUC9 | This work |
| pSA230 | Blocks 3,4,5 in pUC9 | This work |
| pSA233 | Complete HRP gene in pAM18 | This work |

## <u>Table II</u> - Analysis of codon distribution and amino acid content of HRP protein

The distribution of codons in the first reading frame is:

| | | | | | | | |
|---|---|---|---|---|---|---|---|
| TTT | Phe | 9 | (2.9) | TAT | Tyr | 1 | (0.3) |
| TTC | Phe | 11 | (3.6) | TAC | Tyr | 4 | (1.3) |
| TTA | Leu | 3 | (1.0) | TAA | End | 1 | (0.3) |
| TTG | Leu | 2 | (0.6) | TAG | End | 0 | (0.0) |
| TCT | Ser | 2 | (0.6) | TGT | Cys | 3 | (1.0) |
| TCC | Ser | 3 | (1.0) | TGC | Cys | 5 | (1.6) |
| TCA | Ser | 7 | (2.3) | TGA | End | 0 | (0.0) |
| TCG | Ser | 7 | (2.3) | TGG | Trp | 1 | (0.3) |
| | | | | | | | |
| CTT | Leu | 4 | (1.3) | CAT | His | 2 | (0.6) |
| CTC | Leu | 5 | (1.6) | CAC | His | 1 | (0.3) |
| CTA | Leu | 16 | (5.2) | CAA | Gln | 6 | (1.9) |
| CTG | Leu | 5 | (1.6) | CAG | Gln | 7 | (2.3) |
| CCT | Pro | 1 | (0.3) | CGT | Arg | 7 | (2.3) |
| CCC | Pro | 2 | (0.6) | CGC | Arg | 4 | (1.3) |
| CCA | Pro | 9 | (2.9) | CGA | Arg | 6 | (1.9) |
| CCG | Pro | 5 | (1.6) | CGG | Arg | 2 | (0.6) |
| | | | | | | | |
| ATT | Ile | 3 | (1.0) | AAT | Asn | 11 | (3.6) |
| ATC | Ile | 10 | (3.2) | AAC | Asn | 16 | (5.2) |
| ATA | Ile | 0 | (0.0) | AAA | Lys | 2 | (0.6) |
| ATG | Met | 4 | (1.3) | AAG | Lys | 4 | (1.3) |
| ACT | Thr | 7 | (2.3) | AGT | Ser | 1 | (0.3) |
| ACC | Thr | 5 | (1.6) | AGC | Ser | 5 | (1.6) |
| ACA | Thr | 6 | (1.9) | AGA | Arg | 1 | (0.3) |
| ACG | Thr | 7 | (2.3) | AGG | Arg | 1 | (0.3) |

```
GTT Val  2 (0.6)        GAT Asp 10 (3.2)
GTC Val  6 (1.9)        GAC Asp 11 (3.6)
GTA Val  7 (2.3)        GAA Glu  1 (0.3)
GTG Val  2 (0.6)        GAG Glu  6 (1.9)
GCT Ala  5 (1.6)        GGT Gly  3 (1.0)
GCC Ala  5 (1.6)        GGC Gly  4 (1.3)
GCA Ala  8 (2.6)        GGA Gly  8 (2.6)
GCG Ala  5 (1.6)        GGG Gly  2 (0.6)
```

```
The codons represent 309 amino acids:
Ala  23  (7.4)                Leu  35  (11.3)
Arg  21  (6.8)                Lys   6  ( 1.9)
Asn  27  (8.7)                Met   4  ( 1.3)
Asp  21  (6.8)                Phe  20  ( 6.5)
Cys   8  (2.6)                Pro  17  ( 5.5)
Gln  13  (4.2)                Ser  25  ( 8.1)
Glu   7  (2.3)                Thr  25  ( 8.1)
Gly  17  (5.5)                Trp   1  ( 0.3)
His   3  (1.0)                Tyr   5  ( 1.6)
Ile  13  (4.2)                Val  17  ( 5.5)
End   1  (0.3)
```

```
Acidic    (Asp + Glu)              28  ( 9.1)
Basic     (Arg + Lys)              27  ( 8.7)
Aromatic  (Phe + Trp + Tyr)        26  ( 8.4)
Hydrophobic (Aromatic + Ile +
  Leu + Met + Val)                 95  (30.7)
```

```
Molecular Weight = 33922
```

## Claims

1. A synthetic DNA sequence which codes for the HRP C isoenzyme or a conservative variant thereof or a peptide comprising at least one epitope or functional domain thereof.

2. A sequence as claimed in claim 1, which codes for the HRP C isoenzyme or for a conservative variant thereof which generates enhanced luminescence by the catalysis of a substrate such as luminol in the presence of an enhancer of light output.

3. A sequence as claimed in claim 1 or claim 2, wherein the sequence is substantially 940 base pairs and comprises a region coding for the HRP C isoenzyme of substantially 927 base pairs.

4. A sequence as claimed in any one of claims 1 to 3, which includes a single SphI restriction site at its 5' end and a single EcoRV restriction site at its 3' end.

5. A sequence as claimed in any one of claims 1 to 4, which includes spaced single restriction sites for SphI, EcoRI, NarI, DraI, NcoI and EcoRV.

6. A sequence as claimed in claim 5, which includes spaced single restriction sites for the following enzymes in the following order starting at the 5' end:
SphI, NheI, EcoRI, PstI, BstEI, NarI, BalI, RsaI, DraI, BclI, NcoI, EccRV>

7. A sequence as claimed in any one of claims 1 to 5, having a restriction map substantially as shown in Figure 4.

8. A sequence as claimed in any one of claims 1 to 7, having substantially the base pair sequence shown in Figure 6.

9. A sequence as claimed in claim 1, which codes for a peptide comprising at least one epitope or functional domain thereof, said peptide being substantially:-
Gly His Thr Phe Gly Lys Asn Gln Cys Arg Phe Ile
Met Asp Arg Leu Tyr Asn Phe Ser Asn Thr Gly Lue
Pro

10. A vector containing the sequence of any one of claims 1 to 9.

11. A vector as claimed in claim 10, which is based on the plasmid pUC9.

12. A vector as claimed in claim 10, which is based on the plasmid pAM18.

13. A method of making the HRP C isoenzyme or a conservative variant thereof or a peptide comprising at least one epitope or functional domain thereof, which method comprises introducing a vector as claimed in any one of claims 10 to 12 into a host cell and recovering the isoenzyme or peptide therefrom.

14. A method of making the synthetic DNA sequence of any one of claims 1 to 9, which method comprises:
- synthesising a series of oligonucleotides which contain between them all the genetic material of the desired sequence and which have overlapping ends capable of annealing to corresponding ends of neighbouring oligonucleotides in the series
- forming blocks of DNA by mixing two or more neighbouring oligonucleotides of the series under conditions to cause corresponding ends to hybridise, and converting single-stranded to double stranded regions therein by the use of a polymerase enzyme
- joining the blocks of DNA together to form the desired synthetic DNA sequence.

Fig. 1

Fig. 2

Fig. 3

Fig. 4

Fig. 5

```
                                  35                                            65
CAG TTA ACG CCG ACT TTC TAC GAT AAC AGC TGT CCA AAC GTC TCG AAT ATT GTT CGC GAT
Gln Leu Thr Pro Thr Phe Tyr Asp Asn Ser Cys Pro Asn Val Ser Asn Ile Val Arg Asp

                                  95                                           125
ACC ATC GTG AAT GAG CTG CGG TCC GAT CCA CGT ATT GCA GCT AGC ATC CTT CGT CTA CAT
Thr Ile Val Asn Glu Leu Arg Ser Asp Pro Arg Ile Ala Ala Ser Ile Leu Arg Leu His

                                  155                                          185
TTT CAT GAC TGC TTT GTA AAC GGA TGT GAC GCG TCC ATC CTC CTG GAC AAT ACA ACA AGC
Phe His Asp Cys Phe Val Asn Gly Cys Asp Ala Ser Ile Leu Leu Asp Asn Thr Thr Ser

                                  215                                          245
TTC CGA ACT GAG AAA GAC GCA TTC GGG AAC GCG AAT TCG GCC CGG GGC TTT CCA GTC ATC
Phe Arg Thr Glu Lys Asp Ala Phe Gly Asn Ala Asn Ser Ala Arg Gly Phe Pro Val Ile

                                  275                                          305
GAT CGC ATG AAG GCT GCA GTC GAG TCG GCC TGC CCG AGA ACT GTT TCG TGT GCA GAC CTG
Asp Arg Met Lys Ala Ala Val Glu Ser Ala Cys Pro Arg Thr Val Ser Cys Ala Asp Leu

                                  335                                          365
CTG ACC ATC GCA GCC CAG CAA TCG GTC ACC CTA GCC GGC GGG CCC TCA TGG CGA GTA CCA
Leu Thr Ile Ala Ala Gln Gln Ser Val Thr Leu Ala Gly Gly Pro Ser Trp Arg Val Pro

                                  395                                          425
TTA GGA CGT CGT GAC TCC CTA CAG GCT TTC CTA GAT CTA GCT AAT GCA AAT CTA CCG GCG
Leu Gly Arg Arg Asp Ser Leu Gln Ala Phe Leu Asp Leu Ala Asn Ala Asn Leu Pro Ala

                                  455                                          485
CCA TTT TTC ACT CTA CCA CAA CTT AAG GAT TCA TTT CGT AAC GTA GGC CTG AAC CGC TCG
Pro Phe Phe Thr Leu Pro Gln Leu Lys Asp Ser Phe Arg Asn Val Gly Leu Asn Arg Ser

                                  515                                          545
AGT GAT CTA GTA GCT CTT TCT GGT GGC CAC ACT TTT GGA AAG AAC CAG TGC CGA TTC ATC
Ser Asp Leu Val Ala Leu Ser Gly Gly His Thr Phe Gly Lys Asn Gln Cys Arg Phe Ile

                                  575                                          605
ATG GAC CGA TTG TAC AAC TTT TCA AAC ACT GGA CTA CCG GAT CCC ACG CTC AAT ACG ACC
Met Asp Arg Leu Tyr Asn Phe Ser Asn Thr Gly Leu Pro Asp Pro Thr Leu Asn Thr Thr

                                  635                                          665
TAC CTA CAG ACA CTC CGC GGA CTA TGC CCT TTA AAC GGA AAC CTA AGC GCG CTA GTC GAC
Tyr Leu Gln Thr Leu Arg Gly Leu Cys Pro Leu Asn Gly Asn Leu Ser Ala Leu Val Asp

                                  695                                          725
TTT GAC CTA CGT ACG CCA ACA ATC TTC GAC AAC AAG TAC TAT GTA AAT CTA GAG GAA CAA
Phe Asp Leu Arg Thr Pro Thr Ile Phe Asp Asn Lys Tyr Tyr Val Asn Leu Glu Glu Gln

                                  755                                          785
AAA GGA CTT ATT CAA TCT GAT CAG GAG CTC TTC TCA TCA CCG AAT GCA ACA GAC ACG ATC
Lys Gly Leu Ile Gln Ser Asp Gln Glu Leu Phe Ser Ser Pro Asn Ala Thr Asp Thr Ile

                                  815                                          845
CCA CTA GTG CGT AGC TTT GCG AAC TCA ACA CAA ACT TTC TTC AAT GCA TTC GTA GAG GCC
Pro Leu Val Arg Ser Phe Ala Asn Ser Thr Gln Thr Phe Phe Asn Ala Phe Val Glu Ala

                                  875                                          905
ATG GAT CGA ATG GGA AAT ATC ACG CCA CTC ACG GGT ACC CAA GGT CAG ATC AGG TTG AAC
Met Asp Arg Met Gly Asn Ile Thr Pro Leu Thr Gly Thr Gln Gly Gln Ile Arg Leu Asn

TGC CGA GTC GTA AAC TCG AAC TCA TAA
Cys Arg Val Val Asn Ser Asn Ser End
```

Fig. 6

| | **DOCUMENTS CONSIDERED TO BE RELEVANT** | | EP 88306222.6 |
|---|---|---|---|

| Category | Citation of document with indication, where appropriate, of relevant passages | Relevant to claim | CLASSIFICATION OF THE APPLICATION (Int. Cl.4) |
|---|---|---|---|
| D,A | EUROPEAN JOURNAL OF BIOCHEMISTRY, vol. 96, no. 1, May 1979, Berlin, Heidelberg, New York<br><br>K.G.WELINDER "Amino acid sequence studies of horseradish peroxidase." pages 483-502<br><br>* Totality *<br><br>-- | 1,9 | C 12 N 15/00<br>C 07 H 21/04<br>C 12 N  9/08 |
| D,A | THE JOURNAL OF BIOLOGICAL CHEMISTRY, vol. 241, no. 9, May 10, 1966, Baltimore, USA<br><br>L.M.SHANNON et al. "Peroxidase Isozymes form Horseradish Roots." pages 2166-2172<br><br>* Totality *<br><br>-- | 1,13 | |
| A | EP - A2 - 0 179 486 (SUNTORY LIMITED)<br><br>* Abstract *<br><br>-- | 13 | **TECHNICAL FIELDS SEARCHED (Int. Cl.4)** |
| A | US - A - 4 328 312 (TSURUMI et al.)<br><br>* Abstract *<br><br>---- | 13 | C 12 N<br>C 07 H |

The present search report has been drawn up for all claims

| Place of search | Date of completion of the search | Examiner |
|---|---|---|
| VIENNA | 17-10-1988 | WOLF |